# DEMANDE DE BREVET EUROPEEN

(11) **EP 2 410 327 A1**
(43) Date de publication de la demande: **25.01.2012**
(21) Numéro de dépôt: 11290296.0
(22) Date de dépôt: 29.06.2011
(51) Int. Cl.: G01N 33/18, G01N 19/00

(54) **Dispositif de détection de polluants dans un milieu fluide, notamment d'hydrocarbures ou de composés organiques dans un milieu aqueux**

(30) Priorité: 20.07.2010 FR 1003045
(71) Demandeur: IFP Energies nouvelles, 92852 Rueil-Malmaison Cedex (FR)
(72) Inventeur: Pasquier, David, 69006 Lyon (FR); Benoit, Yves, 95480 Pierrelaye (FR); Durand, Isabelle, 92500 Rueil Malmaison (FR); Robert, Valérie, 92500 Rueil Malmaison (FR); Le Roux, Françoise, 92500 Rueil Malmaison (FR)

(57) **Abrégé**

La présente invention concerne un dispositif de détection de polluants dans un milieu fluide, notamment dans un milieu aqueux, comprenant un corps (10) logeant, sous contrainte mécanique, un élément sensible aux polluants (28) par absorption desdits polluants.

Selon l'invention, l'élément sensible (28) comprend une matière à base de polymères dont les propriétés mécaniques varient en fonction de l'absorption des polluants et en ce que le dispositif de détection comprend un moyen de mesure (38) de la variation de l'une au moins desdites propriétés.

## Description

La présente invention se rapporte à un dispositif de détection de polluants dans un milieu fluide.

Elle concerne plus particulièrement un dispositif pour détecter une pollution à base d'hydrocarbures ou de composés organiques (alcools, phénols, éthers,...) dans un milieu aqueux, tel qu'un milieu marin, des nappes phréatiques, des cours d'eau, des effluents aqueux industriels,...

Il est déjà connu, notamment par le document EP 0 620 436 du demandeur, un dispositif destiné à détecter des traces de produits hydrocarbonés contenues dans un milieu aqueux.

Ce dispositif comprend un corps logeant un élément sensible, sous forme d'une bande d'un film de mince épaisseur, au contact du milieu aqueux et qui est interposé, sous compression, entre deux contacteurs électriques.

Ce film, lorsqu'il est au contact d'hydrocarbures, les absorbe et flue au fur et à mesure de l'absorption de ces hydrocarbures. Au terme d'un délai bref, le fluage du film est tel que le contact électrique est établi entre les contacteurs. Ce contact déclenche des alarmes (visuel, sonore, radio, ...) permettant de signaler une pollution du milieu aqueux ou de générer des opérations permettant de combattre cette pollution, comme une projection d'un produit absorbant ou dispersant.

Ce dispositif bien que donnant satisfaction présente des inconvénients non négligeables.

En effet, la détection de pollution par ce dispositif n'est effective qu'au bout d'un temps déterminé qui est notamment fonction de la quantité d'hydrocarbures absorbée, de l'épaisseur et de la composition du film. Par cela, durant la période où le film est en train de fluer sans que le contact électrique ne soit établi, le milieu aqueux continue d'être pollué sans que les alarmes ne soient déclenchées ou que des actions correctives ne soient entreprises.

Dans des cas extrêmes, ce retard dans la détection de pollution peut avoir des conséquences néfastes et parfois irréversibles en termes d'impacts environnementaux, comme la destruction partielle de la faune et la flore aquatique ou la souillure de rivages.

De plus, ce dispositif ne peut donner que des informations binaires sur la présence ou non de polluants sans pour cela pouvoir estimer l'étendue de la pollution. Les alarmes seront donc les mêmes pour une pollution de grande envergure ou une pollution minime.

Ceci peut entrainer des actions de lutte contre cette pollution qui peuvent être disproportionnées, notamment lorsque cette pollution est minime.

Ce capteur de type binaire repose sur un fonctionnement de type "tout ou rien", ce qui a pour inconvénient d'être plus particulièrement adapté à la prévention de pollutions massives, notamment la venue de phases flottantes, d'émulsions ou de polluants présents sous forme particulaire.

La présente invention se propose de remédier aux inconvénients mentionnés ci-dessus grâce à un dispositif de détection rapide qui permet également de signaler une pollution de manière quantitative tout en étant plus particulièrement adapté à la détection de polluants dissous en phase aqueuse à faibles concentrations.

A cet effet, la présente invention concerne un dispositif de détection de polluants dans un milieu fluide, notamment dans un milieu aqueux, comprenant un corps logeant, sous contrainte mécanique, un élément sensible aux polluants par absorption desdits polluants, caractérisé en ce que l'élément sensible comprend une matière à base de polymères dont les propriétés mécaniques varient en fonction de l'absorption des polluants et en ce que le dispositif comprend un dispositif de mesure de la variation de l'une au moins desdites propriétés.

Le dispositif peut comprendre un dispositif de mesure de la variation de l'allongement dudit élément.

Le dispositif peut comprendre des moyens de contraintes mécanique de traction sur l'élément sensible.

L'élément sensible peut être fixé, par une des extrémités, à une partie fixe du dispositif et, par l'autre de ses extrémités, à une partie mobile soumise à une force de traction.

L'élément sensible peut être au moins un film de mince épaisseur d'environ 10 à 300 micromètres.

L'élément sensible peut être majoritairement composé d'élastomères et ces élastomères peuvent être des élastomères thermoplastiques, de type styrène-butadiène styrène ou styrène éthylène butylène styrène, de type polyuréthanne thermoplastique, des élastomères polyétherester, ou des élastomères réticulés de type EPDM ou de type silicone.

L'élément sensible peut être sous la forme d'un ruban rectangulaire ou d'un anneau.

Les moyens pour réaliser une contrainte mécanique de traction peuvent comprendre un poids.

Les moyens pour réaliser une contrainte mécanique de traction peuvent comprendre un ressort.

Les moyens pour réaliser une contrainte mécanique de traction peuvent comprendre une lame de torsion.

Le dispositif de mesure peut comprendre un curseur relié à la partie mobile soumise à l'effort de traction, et une tige sensitive fixe coopérant avec ledit curseur.

Le dispositif de mesure peut être relié à un dispositif de traitement de signal de mesure.

La variation de l'allongement de l'élément sensible peut résulter de la variation des propriétés mécaniques de cet élément lors de l'absorption de polluants.

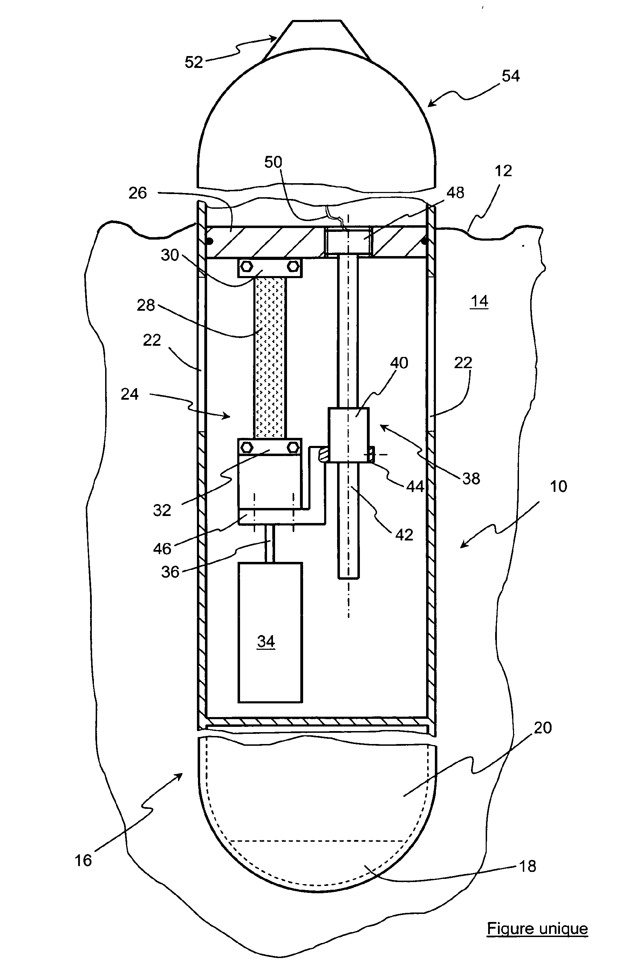

Les autres caractéristiques et avantages de l'invention apparaîtront à la lecture de la description donnée ci-après en se référant à la figure unique qui montre un dispositif de détection de polluants selon l'invention.

La description qui va suivre se fera en relation avec un dispositif de détection de polluants dans un milieu aqueux mais n'écarte en aucune manière un tel dispositif pour la détection d'une pollution gazeuse dans un milieu gazeux, comme l'atmosphère ou une zone insaturée des sols.

Sur la figure annexée, le dispositif de détection de polluants est sous la forme d'un corps creux 10, ici de forme allongée et fermé à ses deux extrémités, flottant sur la surface 12 du milieu aqueux 14, ici de l'eau de mer.

Bien, entendu et cela sans sortir du cadre de l'invention, il peut être prévu que le dispositif dans son entier soit complètement immergé sous l'eau tout en étant situé avantageusement à proximité de la surface 12. Ceci permet de pouvoir également détecter des polluants, notamment des hydrocarbures, qui sont dissous dans cette eau.

Ce corps comprend, en partie basse immergée 16, un lest 18 ainsi qu'une poche d'air 20, étanche au milieu aqueux, qui permettent de maintenir l'ensemble du dispositif en position verticale et en situation de flottabilité.

Ce corps comprend également des ouvertures latérales 22, réparties circonférentiellement tout au long de la paroi périphérique du corps 10, au dessus de la poche d'air 20 et dessous de la surface 12, en considérant la figure.

Ces ouvertures, qui peuvent avoir une forme quelconque (rectangulaire, oblongue, ovale, ...), permettent l'introduction de l'eau à l'intérieur d'un espace 24 du corps 10 délimité par la poche 20 et, en partie supérieure du corps, par un bouchon étanche 26 monté fixement à l'intérieur de ce corps.

Dans cet espace rempli d'eau est logé un élément 28 sensible aux polluants. Cet élément est sous la forme d'au moins une bande d'un film de mince épaisseur, ici une seule bande, qui est fixée par l'une de ses extrémités à un mors fixe 30 porté par le bouchon 26 et par l'autre de ses extrémités à un mors mobile 32.

Cette bande est avantageusement sous la forme d'une bande rectangulaire mais peut être sous la forme d'un anneau.

Préférentiellement, sa matière constitutive est à base de polymères et en particulier d'élastomères, notamment des élastomères thermoplastiques, de type styrène-butadiène styrène ou styrène éthylène butylène styrène, de polyuréthanne thermoplastique, d'élastomères polyétherester, d'élastomère réticulé (EPDM, silicone,...),...

Cette matière a pour avantages de présenter non seulement des propriétés mécaniques qui permettent de répondre à l'utilisation dans le dispositif mais aussi une absorption sélective des composés polluants par rapport à l'eau, ce qui garantit une réponse à des teneurs faibles en polluant.

La bande constituée de cette matière a donc pour particularité de modifier ses propriétés mécaniques en fonction de la quantité et/ou de la nature des polluants absorbés.

Dans le cadre de la description de l'exemple de l'invention qui va suivre, il est entendu par propriété mécanique la résistance à la traction de cette bande qui va se concrétiser par une variation de son allongement lors de la variation de cette résistance.

Bien entendu et cela sans sortir du cadre de l'invention, tout autre propriété, comme la résistance à la compression se concrétisant par une variation d'épaisseur de la bande, peut être également envisagée.

Pour pouvoir ainsi mesurer la variation de sa résistance à la traction, la bande 28 est maintenue sous contrainte mécanique de traction continue en exerçant un effort sur le mors mobile 32.

Sur l'exemple illustré à la figure, cet effort de traction résulte de la mise ne place d'un poids 34 relié à ce mors mobile avantageusement par un tirant 36.

Ce poids peut être remplacé par tout autre moyen de traction, comme un ressort ou une lame de torsion.

Le mors mobile 32 est relié à un moyen étanche de mesure de déplacement 38, en particulier de micro déplacement, qui permet de mesurer la modification ou la variation de la longueur de quelques millimètres de la bande.

A titre d'exemple, ce moyen de mesure comprend un curseur mobile tubulaire 40 coulissant le long d'une tige sensitive 42 fixe. Le curseur 40 est relié au mors mobile 32 par l'intermédiaire d'un doigt 44 porté par un plateau 46 fixé sur ce mors par tous moyens, comme par vissage, et la tige 42 est reliée au bouchon 26 par tous moyens connus, comme également par vissage 48.

Ainsi, tout déplacement du curseur 40 le long de la tige engendre un signal électrique qui est véhiculé par un conducteur électrique 50 vers un dispositif de traitement 52 de ce signal. Préférentiellement, ce dispositif de traitement est porté par la partie supérieure 54 du corps 10, qui est avantageusement situé au dessus de la surface 12 de l'eau ou dans un compartiment hermétique, notamment lorsque le dispositif de détection est complètement immergé.

Un type de ces moyens de mesure de déplacement est plus connu sous la dénomination de capteur LVDT commercialisé par la société Micro Epsilon.

Le dispositif de traitement permet d'évaluer directement ou indirectement (via une unité de calcul par exemple) la présence d'une pollution ainsi que le taux de pollution du milieu aqueux qui est en contact avec la bande. Suite à cela, des alarmes (visuelles, sonores, radios, ...) peuvent être transmises de façon filaire, par ondes radio, par module GSM, ...pour signaler une pollution du milieu aqueux et/ou des opérations permettant de combattre ou réduire cette pollution, comme une projection d'un produit absorbant ou dispersant, peuvent être mises en action.

Bien entendu, l'homme du métier configurera le dimensionnel de bande, notamment son épaisseur, ainsi que la masse du poids 34 et du mors mobile 32 pour que la force exercée par l'eau sur ces éléments ne vienne pas contrecarrer l'effort de traction appliqué à la bande 28 et pour que la contrainte mécanique résultante sur la bande n'entraîne pas un fluage de celle-ci en l'absence de pollution.

Ainsi, le dispositif de détection de polluants décrit ci-dessus est basé sur la modification des propriétés mécaniques de la bande résultant de l'absorption de polluants et sur la mesure en continu de la déformation de cette bande sous l'effet de la contrainte de traction.

En effet, lorsque cette bande polymère est en contact avec une eau polluée par des hydrocarbures, cette bande les absorbe en les concentrant en raison essentiellement de son affinité pour les molécules d'hydrocarbures. Cette propriété de concentration permet ainsi de détecter de très faibles masses de polluants.

En conséquence, cette bande augmente de volume par gonflement aux hydrocarbures, ce qui modifie ses propriétés mécaniques.

Plus particulièrement, le module de traction (ou module de Young), qui reflète la déformation en traction d'un élément en fonction de la force qui lui est appliqué, est changé. Par cela, il est constaté que pour une même force appliquée à la bande, l'allongement de cette dernière est modifié en fonction de l'absorption d'hydrocarbures.

Il est à noter que, compte tenu de la faible concentration en hydrocarbures qu'il est souhaité détecter, la bande a une épaisseur très fine, de l'ordre de 10 à 300µm (micromètre), de façon à pouvoir assurer une détection fine de tout allongement de cette bande ainsi qu'une réponse rapide dû à la rapidité d'absorption des hydrocarbures par la bande.

Ainsi, la force appliquée par le poids 34 est adaptée à la section de la bande et au matériau la constituant, de façon que l'allongement cette bande sous l'effet de la charge reste dans le domaine d'élasticité du matériau de la bande.

En fonctionnement, le dispositif est plongé dans l'eau pour qu'il se trouve dans la configuration de la figure. L'eau pénètre dans l'espace 24 au travers des ouvertures 22 et est au contact avec toutes les faces de la bande 28, qui est en contrainte mécanique de traction sous l'effet du poids 34.

Au préalable, le moyen de mesure de déplacement 38 a été calibré avec une eau non polluée d'une manière telle que le curseur 40 se trouve dans une position nominale correspondante à un allongement nominal L₀ de la bande sous traction.

En cas de contact d'une eau polluée avec la bande, les hydrocarbures contenus dans cette eau sont absorbés par cette bande.

Cette absorption a pour résultat d'augmenter son volume tout en modifiant ainsi sous l'effet du gonflement ses propriétés mécaniques, notamment le module de Young.

Suite à cela, l'allongement (L) de la bande 28 sous l'effet de la traction générée par le poids 34 est modifié, ici augmenté, par rapport à sa calibration.

L'allongement supplémentaire L-L₀ traduit le fluage de la bande sous l'effet conjoint du gonflement et de la contrainte mécanique.

Cet allongement varie donc dans le temps et la cinétique d'allongement - c'est-à-dire la pente de la courbe L-L₀=f(t) - est proportionnelle à la quantité d'hydrocarbures absorbée et va de l'allongement nominal L₀ où l'eau n'est pas polluée jusqu'à la rupture de la bande.

Cette variation d'allongement est transmise, via le mors mobile 32, le plateau 46 et le doigt 44, au curseur 40 qui se déplace le long de la tige sensitive 42. Ce déplacement engendre donc un signal électrique qui est transmis par le conducteur 50 au dispositif de traitement de signal 52 qui déclenche les actions répondant à cette détection de polluants.

Par cela, il est possible non seulement de détecter le début d'une pollution dés que le curseur 40 a quitté sa position nominale mais aussi d'évaluer l'importance de cette pollution et son évolution et cela en fonction de la pente du déplacement du curseur en fonction du temps à partir de sa position nominale.

## Revendications

1. Dispositif de détection de polluants dans un milieu fluide, notamment dans un milieu aqueux, comprenant un corps (10) logeant, sous contrainte mécanique, un élément sensible aux polluants (28) par absorption desdits polluants, **caractérisé en ce que** l'élément sensible (28) comprend une matière à base de polymères dont les propriétés mécaniques varient en fonction de l'absorption des polluants et **en ce que** le dispositif de détection comprend un moyen de mesure (38) de la variation de l'une au moins desdites propriétés.

2. Dispositif selon la revendication 1, **caractérisé en ce qu'**il comprend un moyen de mesure de la variation de l'allongement dudit élément.

3. Dispositif selon la revendication 1 ou 2, **caractérisé en, ce qu'**il comprend des moyens de contraintes mécanique de traction sur l'élément sensible (28).

4. Dispositif de détection de polluants selon la revendication 3, **caractérisé en ce que** l'élément sensible (28) est fixé, par une des extrémités, à une partie fixe (30) du dispositif et, par l'autre de ses extrémités, à une partie mobile (32) soumise à une force de traction.

5. Dispositif de détection de polluants selon l'une des revendications précédentes, **caractérisé en ce que** l'élément sensible (28) comprend au moins un film de mince épaisseur d'environ 10 à 300 micromètres.

6. Dispositif de détection de polluants selon l'une des revendications précédentes, **caractérisé en ce que** l'élément sensible (28) est majoritairement composé d'élastomères.

7. Dispositif de détection de polluants selon la revendication 6, **caractérisé en ce que** les élastomères sont des élastomères thermoplastiques, de type styrène-butadiène styrène ou styrène éthylène butylène styrène, de type polyuréthanne thermoplastique, des élastomères polyétherester, ou des élastomères réticulés de type EPDM ou de type silicone.

8. Dispositif de détection de polluants selon l'une des revendications précédentes, **caractérisé en ce que** l'élément sensible (28) est sous la forme d'un ruban rectangulaire.

9. Dispositif de détection de polluants selon l'une des revendications précédentes, **caractérisé en ce que** l'élément sensible (28) est sous la forme d'un anneau.

10. Dispositif de détection de polluants selon l'une des revendications précédentes, **caractérisé en ce que** les moyens pour réaliser une contrainte mécanique de traction comprennent un poids (34).

11. Dispositif de détection de polluants selon l'une des revendications 1 à 9, **caractérisé en ce que** les moyens pour réaliser une contrainte mécanique de traction comprennent un ressort.

12. Dispositif de détection de polluants selon l'une des revendications 1 à 9, **caractérisé en ce que** les moyens pour réaliser une contrainte mécanique de traction comprennent une lame de torsion.

13. Dispositif de détection de polluants selon l'une des revendications précédentes, caractérisé en ce le moyen de mesure (38) comprend un curseur (40) relié à la partie mobile (32) soumise à l'effort de traction, et une tige sensitive fixe (42) coopérant avec ledit curseur.

14. Dispositif de détection de polluants selon l'une des revendications précédentes, **caractérisé en ce que** le moyen de mesure (38) est relié à un dispositif de traitement de signal de mesure (52).
